# EUROPÄISCHE PATENTSCHRIFT

(11) **EP 1 636 168 B1**
(45) Veröffentlichungstag und Bekanntmachung des Hinweises auf die Patenterteilung: **19.03.2014**
(21) Anmeldenummer: 04726962.6
(22) Anmeldetag: 13.04.2004
(51) Int. Cl.: C07C 231/02

(54) **VERFAHREN ZUR KONTINUIERLICHEN HERSTELLUNG VON ALKYLAMINO(METH)ACRYLAMIDEN**
METHOD FOR CONTINUOUSLY PRODUCING ALKYLAMINO(METH)ACRYLAMIDES
PROCEDE DE PRODUCTION CONTINUE D'ALKYLAMINO(METH)ACRYLAMIDES

(30) Priorität: 22.05.2003 DE 10323699
(43) Veröffentlichungstag der Anmeldung: 22.03.2006
(73) Patentinhaber: Evonik Röhm GmbH, 64293 Darmstadt (DE)
(72) Erfinder: SCHLEEP, Volker, 64683 Einhausen (DE); MERTZ, Thomas, 64625 Bensheim (DE)
(86) Internationale Anmeldenummer: PCT/EP2004/003862
(87) Internationale Veröffentlichungsnummer: WO 2004/103952

(56) Entgegenhaltungen:
- EP-A- 0 362 119
- DE-A- 4 027 843

## Beschreibung

### Gebiet der Erfindung

Die Erfindung betrifft ein weiteres kontinuierliches Verfahren zur Herstellung von Alkylamino(meth)acrylamiden (C) durch kontinuierliche Aminolyse von beispielsweise Methyl(meth)acrylat (A) mit Aminen (B) unter Freisetzung von Methanol (D) gemäß folgender Reaktionsgleichung: 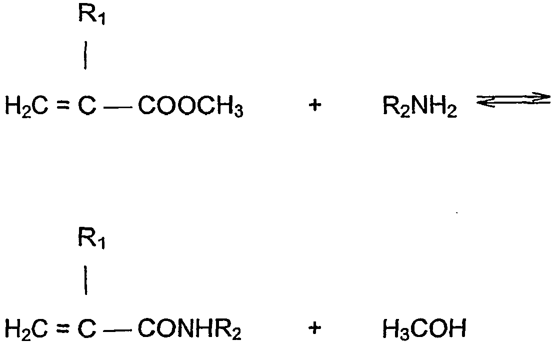 wobei gilt:
- R¹ =: Wasserstoff oder Methyl
- R²: steht für einen linearen, verzweigten oder acyclischen Alkylrest, einen Arylrest, der auch mit ein oder mehreren Alkylgruppen subsituiert sein kann, der lineare, cyclische oder verzweigte Alkylrest kann eine Länge von 2 - 12 Kohlenstoffatomen aufweisen, wie beispielsweise Ethyl Propyl, Isopropyl, Butyl, Isobutyl, tert. Butyl, Pentyl, Hexyl, Heptyl, Octyl, Isooctyl, Nonyl, Decyl, Undecyl bedeuten und kann gegebenenfalls durch
- NR³ R⁴ oder
- OR⁵
   ein- oder mehrfach substituiert sein, dabei kann entweder R³ oder R⁴ die Bedeutung von Wasserstoff annehmen und ferner gilt:
- R³, R⁴ oder R⁵ können entweder gleich oder verschieden sein und eine Alkylgruppe mit 1 - 12 Kohlenstoffatomen darstellen, wie beispielsweise Methyl, Ethyl, Propyl, Isopropyl, Butyl, Isobutyl, tert. Butyl, Pentyl, Hexyl, Heptyl, Octyl, Isooctyl, Nonyl, Decyl, Undecyl oder Wasserstoff.
- R² kann ferner auch

   [(R⁶-0)ₙ] - R⁷

   bedeuten, wobei gilt:
- R⁶ kann eine C₁-C₄-Alkylgruppe sein, die auch verzweigt sein kann, wie beispielsweise Methyl, Ethyl, Propyl, Isopropyl, Butyl, Isobutyl oder tert. Butyl.
   Alkylamido(meth)acrylate
   m: 1 - 4
   R⁷ kann die Methylgruppe oder die Ethylgruppe sein.

Als Amine kommen folgende Verbindungen in Frage:
Dimethylaminoethylamin, Diethylaminoethylamin, Dipropylaminoethylamin, Diisopropylaminoethylamin, Dibutylaminoethylamin, Disobutylaminoehtylamin, Dimethylaminopropylamin, Diethylaminopropylamin, Dipropylaminopropylamin, Diisopropylaminopropylamin, Dibutylaminopropylamin, Disobutylaminopropylamin, Dimethylaminobutylamin, Diethylaminobutylamin, Dipropylaminobutylamin, Diisopropylaminobutylamin, Dibutylaminobutylamin, Disobutylaminobutylamin, Methylamin, Cyclohexylamin, Dimethylaminohexylamin, Diethylaminohexylamin.

Besonders bevorzugt ist neben Dimethylaminopropylamin, Dimethylaminoethylamin, Dimethylaminobutylamin, Dimethylaminopentylamin und Dimethylaminohexylamin.

### Stand der Technik

In der Literatur sind viele diskontinuierlich durchgeführte Umesterungsverfahren (Batch-Umesterungsverfahren) in Verbindung mit unterschiedlichen Katalysatoren beschrieben.

Die Suche nach wirtschaftlicheren Verfahren führte zu der Auffindung von kontinuierlichen Umesterungsverfahren, bei denen die Edukte kontinuierlich zugeführt und die Produkte kontinuierlich abgeführt werden. Die kontinuierlichen Umesterungsverfahren haben gegenüber den diskontinuierlichen Umesterungsverfahren folgende Vorteile: Der Prozess ist leichter automatisierbar und kann mit reduziertem Personalbedarf betrieben werden, die Produktqualität ist besser reproduzierbar und weniger schwankend, die Anlagenkapazität erhöht sich aufgrund des Wegfalls des sequenziellen Abarbeitens der einzelnen Herstellungsschritte (Befüllen, Reaktion, Niedrigsiederabtrennung, Produktabtrennung, Entleeren). Der Prozess besitzt eine höhere Raum-Zeit-Ausbeute als ein Batch-Prozess.

Kontinuierliche Umesterungsverfahren sind bekannt.

EP 0 960 877 (Elf Atochem S.A.) beschreibt ein kontinuierliches Verfahren zur Herstellung von Methacrylatestern von Dialkylaminoalkoholen. Man setzt Dialkylaminoalkohole mit im allgemeinen Methyl(meth)acrylat um und erhält das Dialkylaminoalkyl(meth)acrylat nach folgendem Verfahren:
Das Gemisch der Ausgangsstoffe (Methyl(meth)acrylat und Dialkylaminoalkohol) wird zusammen mit einem Tetraalkyltitanat als Umesterungskatalysator (beispielsweise Tetrabutyl-, Tetraethyl- oder Tetra(2-Ethylhexyl)titanat) und mindestens einem Polymerisationsinhibitor (beispielsweise Phenothiazin, tert.-Butylcatechol, Hydrochinonmonomethylether oder Hydrochinon) kontinuierlich einem Rührreaktor zugeführt, wo bei einer Temperatur von 90 -120 °C die Umsetzung zum Dialkylamino(meth)acrylat bei gleichzeitigem kontinuierlichem Abzug des azeotropen Methyl(meth)acrylat/Methanol-Gemisches erfolgt. Das rohe Reaktionsgemisch (Rohester) wird einer ersten Destillationskolonne zugeführt, wobei man bei vermindertem Druck am Kopf der Destillationskolonne einen im wesentlichen katalysatorfreien Strom abzieht und im Sumpf der Destillationskolonne der Katalysator sowie wenig Dialkylaminoalkyl(meth)acrylat abgezogen werden. Der Kopfstrom der ersten Destillationskolonne wird dann einer zweiten Destillationskolonne zugeführt, bei der man unter vermindertem Druck am Kopf einen Strom von niedrigsiedenden Produkten mit wenig Dialkylaminoalkyl(meth)acrylat und im Sumpf einen Strom bestehend aus hauptsächlich Dialkylaminoalkyl(meth)acrylat sowie Polymerisationsinhibitor(en) abzieht, der einer dritten Destillationskolonne zugeführt wird. In der dritten Destillationskolonne wird unter vermindertem Druck eine Rektifikation durchgeführt, in der man am Kopf den gewünschten reinen Dialkylaminoalkyl(meth)acrylatester und im Sumpf im wesentlichen den Polymerisationsinhibitor oder die Polymerisationsinhibitoren abzieht. Der Sumpfstrom der ersten Destillationskolonne wird nach weiterer Aufreinigung mit Hilfe eines Filmverdampfers genauso in den Reaktor zurückgeführt wie der Kopfstrom aus der zweiten Destillationskolonne.

Dieses Verfahren verzichtet auf eine Entwässerung der Alkohole vor dem Einsatz, was zu einer verstärkten Desaktivierung des eingesetzten Tetraalkyltitanats infolge Hydrolyse bis hin zur Bildung unerwünschter Feststoffniederschläge führen kann. Weiterhin verfügt das Verfahren über den Nachteil, dass in der ersten Destillationskolonne der Katalysator im Sumpf bei relativ hohen Temperaturen thermisch beansprucht wird. Dies kann leicht zur Zersetzung des Katalysators führen. Bei diesem Verfahren werden sowohl die nicht umgesetzten Edukte wie auch das Produkt insgesamt zweimal über Kopf rektifiziert. Dies erfordert sehr hohe Energiekosten und insgesamt 4 Rektifikationskolonnen, die zum Teil sehr groß dimensioniert sein müssen. Der Prozeß ist daher mit sehr hohen Investitions- und Betriebskosten belastet.

EP 0 968 995 (Mitsubishi Gas Chemical Comp.) beschreibt ein kontinuierliches Verfahren zur Herstellung von Alkyl(meth)acrylsäureestern unter Verwendung einer Reaktionskolonne. Die Umesterungsreaktion erfolgt dabei direkt in einer Destillationskolonne (d.h. Reaktor und Destillationskolonne zum Abzug des Methyl(meth)acrylat/Methanol-Azeotrops bilden einen Apparat), der die Ausgangsstoffe (Methyl(meth)acrylat und Alkohol) kontinuierlich zugeführt werden. Der notwendige Katalysator, hier ebenfalls bevorzugt eine Titanverbindung, befindet sich in der Destillationskolonne. Im Fall eines homogenen Katalysators wird der Katalysator in die Destillationskolonne kontinuierlich eindosiert. Die Verwendung von homogenen Katalysatoren in einer Destillationskolonne führt jedoch aufgrund eines Spüleffektes durch den flüssigen Rücklauf in der Destillationskolonne zu einem erhöhten Katalysatorbedarf sowie bei Auftreten eines Katalysatorfeststoffniederschlags zur Verschmutzung der Kolonneneinbauten. Im Fall eines heterogenen Katalysators befindet sich der Katalysator in der Reaktionskolonne. Die Positionierung des Katalysators in der Destillationskolonne ist allerdings nachteilig, weil in der Destillationskolonne dann ein erhöhter Druckverlust auftritt und zusätzlich für die regelmäßige Reinigung der Destillationskolonne ein sehr hoher Aufwand betrieben werden muss. Weiterhin können heterogene Katalysatoren beispielsweise infolge unerwünschter Polymerisation desaktivieren.

DE 4 027 843 (Röhm GmbH) beschreibt ein kontinuierliches Verfahren zur Herstellung N-substituierter (Meth)acrylsäureamiden durch Umesterung von Alkylestern der (Meth)acrylsäure mit aliphatischen und aromatischen Aminen. Die Reaktionstemperatur liegt bei > 150°, der Druck beträgt ca. 160 bar. Es wird ohne Katalysator gearbeitet.

### Aufgabe

Aufgabe der vorliegenden Erfindung ist es, ein kontinuierliches Verfahren zur Aminolyse von (Meth)acrylsäuremethylester mit gegenüber Methanol höhersiedenden Aminen zur Verfügung zu stellen, das die Nachteile der beiden vorgenannt beschriebenen Verfahren vermeidet. Unter (Meth)acrylsäureestern oder Alkyl(meth)acrylaten werden im folgenden Ester und Derivate der Acrylsäure und der Methacrylsäure verstanden, wie beispielsweise der Methacrylsäuremethylester oder der Methacrylsäureethylester. Weiterhin soll durch das neue Verfahren ein Produkt zur Verfügung gestellt werden, das in der Qualität besser ist als die bisher auf dem Markt befindlichen. Unter einer besseren Qualität wird ein geringerer Vernetzergehalt oder ein geringerer Gehalt an Additionsprodukten der Amine an die Doppelbindung des Ausgangsesters oder an die Doppelbindung des Produktesters verstanden. Als Vernetzer kann Alkylmethacrylamid gebildet werden. Ferner sollen mit dem neuen Verfahren Amino(meth)acrylate mit möglichst wenig Aufwand und energetisch günstiger (d.h. kostengünstiger) hergestellt werden. Der Personalaufwand zur Bedienung der Anlage soll verringert werden.

### Verfahrensbeschreibung

Das Verfahren ist schematisch in Figur 1 dargestellt.

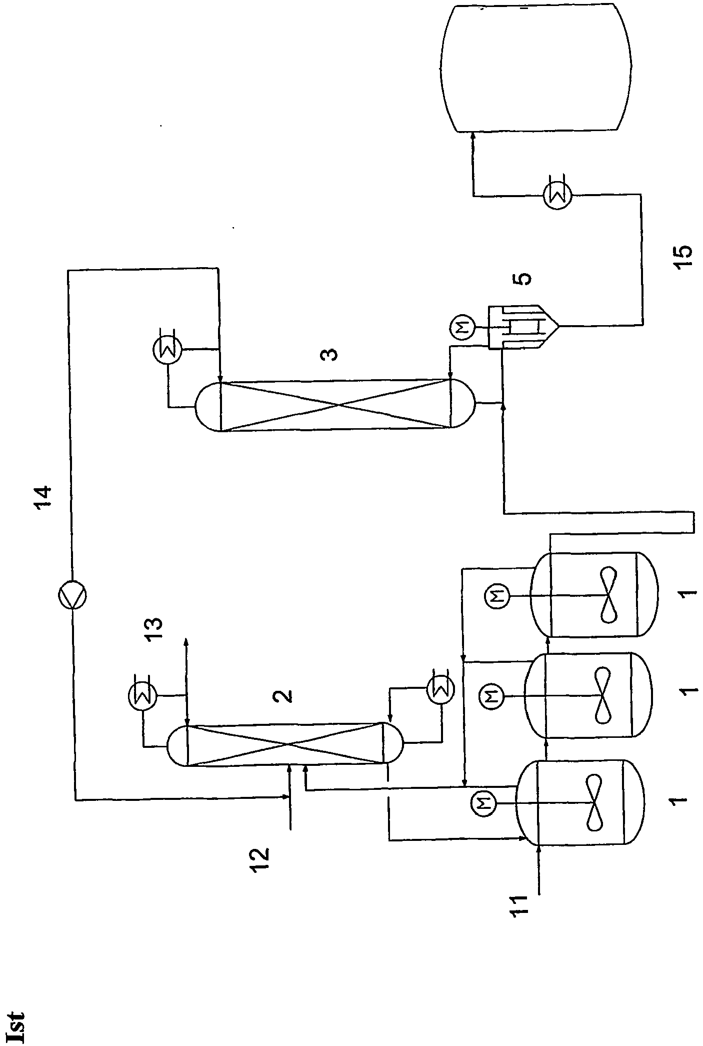

Erläuterungen der Bezugszeichen Figur 1:
1. Reaktionsapparat
2. Azeotrop-Destillationskolonne
3. Niedrigsieder-Destillationskolonne
5. Filmverdampfer
11. Methyl(meth)acrylat- und Katalysator-Feed
12. Amin-Feed
13. Methanol/Methyl(meth)acrylat-Azeotrop
14. Niedrigsieder-Kreislaufstrom
15. Rohprodukt

Das Edukt Methyl(meth)acrylat (MMA, 11) wird kontinuierlich einem geeigneten Reaktionsapparat (1) zugeführt, wobei sowohl ein einzelner Reaktionsbehälter als auch eine Kaskade von mehreren hintereinander geschaltenen Reaktionsbehältern eingesetzt werden kann. Es ist sinnvoll, dass alle Reaktionsbehälter einen Brüdenabzug zur Azeotrop-Destillationskolonne (2) zur Entfernung des bei der Reaktion frei werdenden Methanols besitzen.

Das Amin (12) wird kontinuierlich der Azeotrop-Destillation der Kolonne zur Entwässerung zugeführt.

Das als Katalysator benötigte Tetraalkoxititanat (der Tetraalkoxititanat-Gehalt in Bezug auf eingesetztes MMA beträgt bevorzugt 0,2 - 4 Gew.-%) wird wie der/die Polymerisationsinhibitor/en ebenfalls bevorzugt kontinuierlich in den Reaktionsapparat (1) zudosiert. Als Umesterungskatalysatoren können aber auch alle aus dem Stand der Technik bekannten Umesterungskatalysatoren eingesetzt werden. Als Katalysatoren kommen beispielsweise Zirkonacetylacetonat und weitere 1.3-Diketonate des Zirkons in Frage, ferner können Mischungen aus Alkalimetallcyanaten oder Alkalimetallthiocyanaten und Alkalimetallhalogeniden eingesetzt werden, ferner Zinkverbindungen, beispielsweise Dioctylzinkoxid, Erdalkalimetalloxide oder Erdalkalimetallhydroxide, wie beispielsweise CaO, Ca(OH)₂, MgO, Mg(OH)₂ oder Gemische aus den vorstehend genannten Verbindungen, ferner Alkalimetallhydroxide, Alkalimetallalkoxide und Lithiumchlorid und Lithiumhydroxid, es können auch Gemische aus den vorstehend genannten Verbindungen mit den vorstehend genannten Erdalkaliverbindungen und den Li-Salzen eingesetzt werden, Dialkylzinnoxide, wie beispielsweise Dioctylzinnoxid, Alkalimetallcarbonate, Alkalimetallcarbonate zusammen mit quartären Ammoniumsalzen, wie beispielsweise Tetrabutylammoniumhydroxid oder Hexadecyltrimethylammoniumbromid, ferner Mischkatalysatoren aus Diorganylzinnoxid und Organylzinnhalogenid, saure Ionenaustauscher, Phosphormolybdän-Heteropolysäuren, Titanalkoholate, wie beispielsweise Isopropyltitanat, Chelatverbindungen der Metalle Titan, Zirkonium, Eisen oder Zink mit 1.3-Di-Carbonylverbindungen, Bleiverbindungen, wie beispielsweise Bleioxide, Bleihydroxide, Bleialkoxide, Bleicarbonate oder Bleisalze von Carbonsäuren. Besonders bevorzugt ist ein Katalysatorgemisch aus Dialkylzinnoxid und Alkyltitanat, beispielsweise Dioctylzinnoxid und Isopropyltitanat im Verhältnis ca. 1 : 1 (Gew.-%/Gew.-%). Das Katalysatorgemisch wird in Mengen von 0,1 - 10 Masse-%, bezogen auf das eingesetzte Amin, eingesetzt.

Als Polymerisationsinhibitoren kommen beispielsweise Hydrochinon, 4-Hydroxy-2,2,6,6-tetra-methylpiperidinooxyl oder auch Bis(2-methoxycarbonylpropyl)sulfid in Frage. Hydrochinonmonomethylether in Verbindung mit Sauerstoff in Frage.

Das eingesetzte Amin kann Wasser enthalten. Die Menge an Wasser im eingesetzten Amin liegt im Falle von Amin zwischen 50 und 500 ppm (0,05-0,005 Gew.-%). Das Amin wird vor dem Eintreten in den Reaktionsapparat bevorzugt über die Azeotrop-Kolonne (2) destillativ entwässert. Dabei wird das im Amin enthaltene Wasser über Kopf abgezogen. Zur Vermeidung einer Verunreinigung des Methanol/MMA-Azeotrops (13) mit dem eingesetzten Amin erfolgt die Aufgabe des Amins bevorzugt im unteren Teil der Destillationskolonne (2). Das eingesetzte Amin kann auch auf andere Art und Weise entwässert werden:
- durch eine vorgeschaltete Entwässerungs-Destillationskolonne oder
- durch Behandlung mit einem Entwässerungsmittel, wie beispielsweise ein Molekularsieb,
   oder
- durch ein Membrantrennverfahren, wie beispielsweise eine Pervaporation.

Die Entwässerung ist daher bedeutsam, da das im Amin enthaltene Wasser zur irreversiblen Schädigung des Katalysators (z.B. Tetraalkyltitanat) im Reaktor führen kann. Das im Amin enthaltene Wasser führt zur Bildung von Nebenprodukten und ist daher strikt zu vermeiden. Durch diesen Entwässerungsschritt vermeidet man die Hydrolyse des Katalysators und die damit entstehenden Kosten durch erhöhte Katalysatoreinsatzmengen und durch Probleme mit Feststoffniederschlägen. Außerdem wird die Reinheit des Produktes durch einen verringerten Anteil an Nebenprodukten erhöht.

Die Umsetzung erfolgt im Reaktionsapparat (1) bei einer Temperatur im Bereich zwischen 80 und 160 °C. Bevorzugt ist der Temperaturbereich zwischen 110 und 135 °C. Zur Erhöhung der Reaktionsgeschwindigkeit wird das bei der Reaktion frei werdende Methanol über die Destillationskolonne (2) aus dem Reaktionsgemisch als Azeotrop mit MMA abgezogen (13). Die Reaktionsmischung, welche größtenteils aus dem Produkt-Alkyl(meth)acrylatamid, nicht umgesetztem MMA und Amin sowie geringen Mengen Methanol, dem Katalysator, den Polymerisationsinhibitoren und einem sehr geringen Anteil an Nebenprodukten besteht, wird nach ca. 0,5 - 3 Stunden Reaktorverweilzeit (bevorzugt ist eine Verweilzeit von 0,75 - 1,5 Stunden) einem kontinuierlich betriebenenFallfilmverdampfer (5) zugeführt. Die Brüden des Fallfilmverdampfers (5) werden einer Niedrigsieder-Destillationskolonne (3) zugeführt. Dort erfolgt bei vermindertem Druck, bevorzugt im Bereich von 10-500 mbar, die Abtrennung der in Bezug auf den Produktester niedrigsiedenden Komponenten, vorwiegend Methanol, MMA und nicht umgesetztes Edukt-Amin. Diese werden über den Kopf der Destillationskolonne abgezogen und in den Reaktorbereich oder in die Azeotropkolonne (2) zurückgeführt (14). Durch diesen Kreislaufstrom wird garantiert, dass, bezogen auf den gesamten Prozess, praktisch vollständiger Umsatz in Bezug auf die Edukte MMA und Amin vorliegt.

Der im Ablauf des Fallfilmverdampfers (5) anfallende mit Katalysator, Polymerisationsinhibitor und hochsiedenden Nebenprodukten noch verunreinigte Roh-Amid (15) enthält vorzugsweise > 93 Gew.-% Produktester und wird zur Aufarbeitung einer weiteren Vakuumdestillationsstufe, welche im bevorzugten Druckbereich zwischen 20 und 200 mbar arbeitet, zugeführt. Hier erfolgt die destillative Abtrennung des hochreinen Produktamin als Kopfprodukt.

Die im Prozeß gebildeten Nebenprodukte stellen in Bezug auf das Eduktamin und das Methylmethacrylat hochsiedende Komponenten dar und gelangen somit als Verunreinigung in den Produktester, wodurch die Produktqualität deutlich gesenkt wird. Dieses Problem kann dadurch gelöst werden, dass man zur Abtrennung des Produktamins vom Katalysator und den Polymersiationsinhibitoren sowie den hochsiedenden Nebenprodukten einen Apparat mit schonender Filmverdampfung (5) einsetzt. Als geeignete Apparate sind hierfür Fallfilm-, Dünnschicht- und Kurzwegverdampfer bekannt.

An die Herstellung der Alkylamino(meth)acrylamide kann sich gegebenenfalls noch eine Reindestillationsanlage anschließen, die auch unter vermindertem Druck, beispielsweise bei 500-50 mbar, betrieben werden kann.

Das erfindungsgemäße Verfahren wird durch die nachfolgenden Beispiele näher erläutert, ohne darauf beschränkt zu sein.

### Beispiel: Kontinuierlich beschriebene Aminolyse zu Aminoestern

Zur kontinuierlich beschriebenen Herstellung von N-Dimethylaminopropylmethacrylamid (Aminoester) werden dem
1. Reaktionskessel 235 kg/h MMA/Katalysator-Feed mit einem Anteil von 3,8 Gew.-% Isopropyl-Titanat 3,0 Gew.-% Dioctylzinnoxid der Azeotrop-Destillationskolonne und 244 kg/h N-Dimethylaminopropylamin (DMAPA) zudosiert. Weiterhin floss dem 1. Reaktionskessel über die Azeotrop-Kolonne der Kreislaufrückstrom vom Kopf der Niedrigsieder-Destillationskolonne kontinuierlich zu (195 kg/h mit der Zusammensetzung 78,9 Gew.-% MMA, 2,12 Gew.-% Methanol, 10,1 Gew.-% DMAPA und 8,88 Gew.-% Nebenprodukte). Das molare MMA : DMAPA-Verhältnis im Reaktorzulauf betrug 1,23 : 1. Weiterhin liefen die in der Azeotrop-Kolonne vom Methanol befreiten Brüden der Rührkessel über den Azeotrop-Kolonnen-Sumpf dem
1. Reaktionskessel zu. Unter diesen Reaktionsbedingungen stellte sich eine Reaktionstemperatur von 107°C im 1. Reaktionskessel ein. Der Destillat-Abzug der Azeotrop-Kolonne betrug 117 kg/h mit 56,54 Gew.-% Methanol, 39,12 Gew.-% MMA, 4,02 Gew.-% Isopropanol und 0,5 Gew.-% Nebenprodukte.

Der Ablauf des 1. Reaktionskessels lief in den 2. Reaktionskessel und der Ablauf des 2. Reaktionskessels lief in den 3. Reaktionskessel. Bei einer Verweilzeit von ca. 15 Min. im 1. Reaktionskessel, ca. 30 Min. im 2. Reaktionskessel und ca. 60 Min. im 3. Reaktionskessel wurden folgende Zusammensetzungen in den Reaktoren erhalten.

| | T (°C) | MMA Gew.-(%) | DMAPA Gew.-(%) | Aminoester Gew.-(%) | Methanol Gew.-(%) | Nebenprod. Gew.-(%) |
|---|---|---|---|---|---|---|
| 1. Reaktor | 107 | 62,7 | 10,23 | 22,07 | 0,62 | 4,38 |
| 2. Reaktor | 111 | 55,6 | 10,15 | 27,69 | 0,59 | 5,97 |
| 3. Reaktor | 130 | 46,8 | 4,86 | 41,29 | 1,24 | 5,81 |

Die Brüden der einzelnen Reaktionskessel wurden kontinuierlich der Azeotrop-Kolonne zugeführt.

Der Ablauf des 3. Reaktionskessels lief kontinuierlich dem Dünnschichtverdampfer einer Niedrigsieder-Kolonne zu, in der nicht umgesetztes DMAPA, MMA und Methanol als Destillat (195 kg/h) abgezogen und als Kreisstrom dem 1. Reaktionskessel wieder zugeführt wurden. Der Sumpf-Anlauf des Dünnschichtverdampfers der Niedrigsiederkolonne betrug 426 kg/h und hatte die Zusammensetzung: 93 % Produkt-Aminoester, 0,5 % DMAPA, 0,2 % MMA, 2,15 Gew.-% eines MMA-Amin Adduktes und 4,25 Gew.-% sonstige Nebenprodukte.

## Patentansprüche

1. Verfahren zur **kontinuierlich** betriebenen Herstellung von Alkylamino(meth)acrylamid der **Formel** (C), 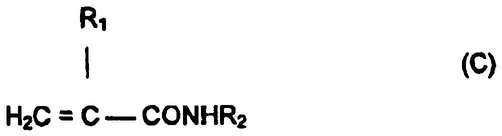 worin R₁ für eine H- oder CH₃-Gruppe und R₂ für einen linearen, verzweigten oder cyclischen Alkylrest oder Arylrest mit 2 bis 12 C-Atomen steht, durch Umsetzung einer Verbindung der Formel (B),
R₂NH₂ (B)
wobei R₂ die oben angegebene Bedeutung besitzt, mit einer Verbindung der Formel (A), 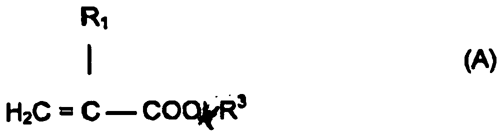 worin R₁ für eine H- oder CH₃-Gruppe steht und R³ Methyl oder Ethyl bedeuten kann, in Gegenwart eines Umesterungskatalysators und in Gegenwart mindestens eines Polymerisationsinhibitors in einer Apparatur zur kontinuierlichen Umsetzung,
**dadurch gekennzeichnet,**
**dass** die Reaktanden einem geeigneten Reaktionsapparat (1) **kontinuierlich** zugeführt werden und dass der bei der Reaktion entstehende Alkohol als azeotropes Methanol/Methyl(meth)acrylat-Gemisch (13) beziehungsweise Ethanol/Ethylacrylat-Gemisch 13 kontinuierlich mit Hilfe einer Destillationskolonne (2) abgezogen wird und außerdem:
- das Reaktion gemisch kontinuierlich aus dem Reaktionsapparat in eine Destillationskolonne (3) bzw. einen Verdampfer (5) geführt wird, wobei durch Destillation unter vermindertem Druck Ober Kopf die leicht flüchtigen Komponenten A, B, Methanol bzw. Ethanol und ein sehr geringer Anteil Produktamid (C) abgezogen und in den Reaktionsapparat zurückgeführt werden und aus dem Sumpf der Kolonne die Produktamid (C) zusammen mit dem Katalysator und den Polymerisationsinhibitoren sowie hochsiedenden Nebenprodukten abgezogen werden;
- der Sumpfstrom (15) aus der Destillationskolonne (3) einer Reindestillation kontinuierlich zugeführt wird.

2. Verfahren nach Anspruch 1,
**dadurch gekennzeichnet,**
**dass** der Brüdenstrom des Verdampfers (5) einer weiteren Destillationskolonne kontinuierlich zugeführt wird, wobei durch Destillation unter vermindertem Druck über Kopf das hochreine Produktamid (C) abgeführt wird und Ober den Sumpf der Katalysator und die Polymerisationsinhibitoren sowie die hochsiedenden Nebenprodukte it einem kleinen Teil Produktamid (C) abgezogen werden.

3. Verfahren nach Anspruch 1,
**dadurch gekennzeichnet,**
**dass** das Amin (B) zur Entwässerung Ober die Destillationskolonne (2) dem Reaktionsapparat zugeführt wird.

4. Verfahren nach Anspruch 1,
**dadurch gekennzeichnet,**
**dass** das molare Verhältnis von Methyl(meth)acrylat bzw. Ethyl(meth)acrylat (EMA) zu Amin im Zulauf zum Reaktor zwischen 1 und 2, vorzugsweise 1,05 -1,15 beträgt.

5. Verfahren nach Anspruch 1,
**dadurch gekennzeichnet,**
**dass** man als Umesterungskatalysator ein Tetryaalkyltitanat einsetzt.

6. Verfahren nach Anspruch 1,
**dadurch gekennzeichnet,**
**dass** der Katalysator in einer Menge von 0,1 - 10 Gew.-%, bezogen auf eingesetztes MMA bzw. EMA, eingesetzt wird.

7. Verfahren nach Anspruch 7,
**dadurch gekennzeichnet,**
**dass** der Katalysator in einer Menge von 0,2 - 7 Gew.-%, bezogen auf eingesetztes MMA bzw. EMA, eingesetzt wird.

8. Verfahren nach Anspruch 1,
**dadurch gekennzeichnet,**
**dass** man als Katalysatormischung eine Mischung aus Dioctylzinnoxid und Isopropyltitanat im Verhältnis 1 : 1 (Gew.-%) anwendet.

9. Verfahren nach Anspruch 8,
**dadurch gekennzeichnet,**
**dass** das Katalysatorgemisch in einer Menge von 0,1 - 10 Gew.-% bezogen auf eingesetztes MMA bzw. EMA, eingesetzt wird.

10. Verfahren nach Anspruch 7,
**dadurch gekennzeichnet,**
**dass** der Katalysator in einer Menge von 0,2 - 7 Gew.-%, bezogen auf eingesetztes MMA bzw. EMA, eingesetzt wird.

11. Verfahren nach Anspruch 1,
**dadurch gekennzeichnet,**
**dass** man als Polymerisationsinhibitor entweder Phenothiazin, Tertiärbutylcatechol, Hydrochlnonmonomethylether, Hydrochinon oder deren Gemische einsetzt, wobei die Menge des Inhibkor zwischen 100 und 5000 ppm, bezogen auf das Reaktionsgemisch, beträgt.

12. Verfahren nach Anspruch 1,
**dadurch gekennzeichnet,**
**dass** man als Polymerisationsinhibitor zusätzlich noch Sauerstoff verwendet.

13. Verfahren nach Anspruch 1,
**dadurch gekennzeichnet,**
**dass** man als Amin vorzugsweise Dimethylaminopropylamin verwendet.

14. Verfahren nach Anspruch 1,
**dadurch gekennzeichnet,**
**dass** der Druck in der ersten Destillationskolonne (3) zwischen 20 und 500 mbar beträgt.

15. Verfahren nach Anspruch 1,
**dadurch gekennzeichnet,**
**dass** die Verweilzeit im Reaktionsapparat zwischen 0,5 und 1,5 Stunden beträgt.

16. Verfahren nach Anspruch 1,
**dadurch gekennzeichnet,**
**dass** der Verdampfer (5) ein Filmverdampfer ist.

## Claims

1. Process for continuously preparing alkylamino-(meth)acrylamide of the formula (C) 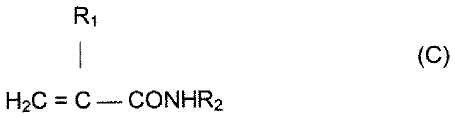 where R₁ is an H or CH₃ group and R² is a linear, branched or cyclic alkyl radical or aryl radical having 2 to 12 carbon atoms, by reacting a compound of the formula (B)
R₂NH₂ (B)
where R² is as defined above with a compound of the formula (A) 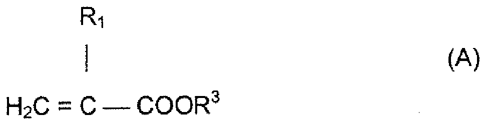 where R₁ is an H or CH₃ group and R³ is methyl or ethyl, in the presence of a transesterification catalyst and in the presence of at least one polymerization inhibitor in an apparatus for continuous reaction,
**characterized in that**
the reactants are fed continuously to a suitable reaction apparatus (1) and **in that** the alcohol formed in the reaction is removed continuously with the aid of a distillation column (2) as an azeotropic methanol/methyl (meth)acrylate mixture (13) or ethanol/ethyl acrylate mixture 13, and also:
- the reaction mixture is conducted continuously out of the reaction apparatus into a distillation column (3) or an evaporator (5) in which the volatile components A, B, methanol or ethanol and a very small fraction of product amide (C) are removed via the top by distillation under reduced pressure and recycled into the reaction apparatus, and the product amides (C) are removed from the bottom of the column together with the catalyst and the polymerization inhibitors, and also high-boiling by-products;
- the bottom stream (15) from the distillation column (3) is fed continuously to a purifying distillation.

2. Process according to Claim 1,
**characterized in that**
the vapour stream of the evaporator (5) is fed continuously to a further distillation column in which the highly pure product amide (C) is removed via the top by distillation under reduced pressure, and the catalyst and the polymerization inhibitors, and also the high-boiling by-products with a small portion of product amide (C) are removed via the bottom.

3. Process according to Claim 1,
**characterized in that**
the amine (B) is dewatered by feeding it to the reaction apparatus via the distillation column (2).

4. Process according to Claim 1,
**characterized in that**
the molar ratio of methyl (meth)acrylate or ethyl (meth)acrylate (EMA) to amine in the feed to the reactor is between 1 and 2, preferably 1.05 - 1.15.

5. Process according to Claim 1,
**characterized in that**
the transesterification catalyst used is a tetraalkyl titanate.

6. Process according to Claim 1,
**characterized in that**
the catalyst is used in an amount of 0.1 - 10% by weight, based on MMA or EMA used.

7. Process according to Claim 6,
**characterized in that**
the catalyst is used in an amount of 0.2 - 7% by weight, based on MMA or EMA used.

8. Process according to Claim 1,
**characterized in that**
the catalyst mixture employed is a mixture of dioctyltin oxide and isopropyl titanate in a ratio of 1 : 1 (% by weight).

9. Process according to Claim 8,
**characterized in that**
the catalyst mixture is used in an amount of 0.1 - 10% by weight, based on MMA or EMA used.

10. Process according to Claim 7,
**characterized in that**
the catalyst is used in an amount of 0.2 - 7% by weight, based on MMA or EMA used.

11. Process according to Claim 1,
**characterized in that**
the polymerization inhibitor used is either phenothiazine, tert-butylcatechol, hydroquinone monomethyl ether, hydroquinone or mixtures thereof,
and the amount of the inhibitor is between 100 and 5000 ppm, based on the reaction mixture.

12. Process according to Claim 1,
**characterized in that**
oxygen is additionally used as a polymerization inhibitor.

13. Process according to Claim 1,
**characterized in that**
the amine used is preferably dimethylaminopropylamine.

14. Process according to Claim 1,
**characterized in that**
the pressure in the first distillation column (3) is between 20 and 500 mbar.

15. Process according to Claim 1,
**characterized in that**
the residence time in the reaction apparatus is between 0.5 and 1.5 hours.

16. Process according to Claim 1,
**characterized in that**
the evaporator (5) is a film evaporator.

## Revendications

1. Procédé pour la production conduite en continu d'alkylamino(méth)acrylamide de formule (C), 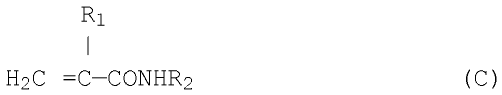 dans laquelle R₁ représente un groupe H ou CH₃ et R₂ représente un radical alkyle linéaire, ramifié ou cyclique ou un radical aryle, ayant de 2 à 12 atomes de carbone, par mise en réaction d'un composé de formule (B),
R₂NH₂ (B)
dans laquelle R² a la signification indiquée ci-dessus, avec un composé de formule (A), 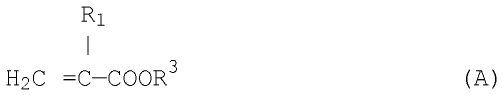 dans laquelle R₁ représente un groupe H ou CH₃ et R³ peut représenter le groupe méthyle ou éthyle, en présence d'un catalyseur de transestérification et en présence d'au moins un inhibiteur de polymérisation, dans un appareil pour la réaction continue,
**caractérisé en ce que**
les partenaires réactionnels sont envoyés en continu à un appareil de réaction (1) approprié et **en ce que** l'alcool formé dans la réaction est évacué en continu sous forme de mélange azéotropique de méthanol/(méth)-acrylate de méthyle (13) ou de mélange azéotropique d'éthanol/acrylate d'éthyle 13 à l'aide d'une colonne de distillation (2) et en outre :
- le mélange réactionnel est envoyé en continu à partir de l'appareil de réaction dans une colonne de distillation (3) ou un évaporateur (5), où les composants très volatils A, B, méthanol ou éthanol et une très petite quantité d'amide (C) produit sont évacués par distillation sous pression réduite, par la tête et renvoyés dans l'appareil de réaction et l'amide (C) produit est déchargé du pied de la colonne conjointement avec le catalyseur et les inhibiteurs de polymérisation ainsi que les sous-produits à haut point d'ébullition ;
- le courant de pied (15) est envoyé en continu à partir de la colonne de distillation (3) à une distillation conduisant à l'état pur.

2. Procédé selon la revendication 1,
**caractérisé en ce que**
le courant de vapeurs de l'évaporateur (5) est envoyé en continu dans une autre colonne de distillation, dans laquelle l'amide très pur (C) produit est évacué par distillation sous pression réduite, par la tête et le catalyseur et les inhibiteurs de polymérisation ainsi que les sous-produits à haut point d'ébullition sont évacués par le pied avec une petite partie de l'amide (C) produit.

3. Procédé selon la revendication 1,
**caractérisé en ce que**
l'amine (B) est envoyée via la colonne de distillation (2) pour la déshydratation à l'appareil de réaction.

4. Procédé selon la revendication 1,
**caractérisé en ce que**
le rapport molaire du (méth)acrylate de méthyle ou du (méth)acrylate d'éthyle (EMA) à l'amine dans le conduit d'arrivée au réacteur est compris entre 1 et 2, de préférence 1,05 - 1,15.

5. Procédé selon la revendication 1,
**caractérisé en ce**
**qu'**on utilise comme catalyseur de transestérification un titanate de tétraalkyle.

6. Procédé selon la revendication 1,
**caractérisé en ce**
**qu'**on utilise le catalyseur en une quantité de 0,1-10 % en poids, par rapport au MMA ou à l'EMA utilisé.

7. Procédé selon la revendication 6,
**caractérisé en ce que**
qu'on utilise le catalyseur en une quantité de 0,2-7 % en poids, par rapport au MMA ou à l'EMA utilisé.

8. Procédé selon la revendication 1,
**caractérisé en ce**
**qu'**on utilise comme mélange de catalyseurs un mélange d'oxyde de dioctylétain et de titanate d'isopropyle en le rapport 1 : 1 (% en poids).

9. Procédé selon la revendication 8,
**caractérisé en ce que**
qu'on utilise le mélange de catalyseurs en une quantité de 0,1 - 10 % en poids, par rapport au MMA ou à l'EMA utilisé.

10. Procédé selon la revendication 7,
**caractérisé en ce que**
qu'on utilise le catalyseur en une quantité de 0,2-7 % en poids, par rapport au MMA ou à l'EMA utilisé.

11. Procédé selon la revendication 1,
**caractérisé en ce**
**qu'**on utilise comme inhibiteur de polymérisation soit de la phénothiazine, du tert-butylcatéchol, de l'éther monométhylique d'hydroquinone, de l'hydroquinone, soit des mélanges de ceux-ci, la quantité de l'inhibiteur étant comprise entre 100 et 5 000 ppm, par rapport au mélange réactionnel.

12. Procédé selon la revendication 1,
**caractérisé en ce**
**qu'**on utilise comme inhibiteur de polymérisation en outre encore de l'oxygène.

13. Procédé selon la revendication 1,
**caractérisé en ce**
**qu'**on utilise comme amine de préférence la diméthylaminopropylamine.

14. Procédé selon la revendication 1,
**caractérisé en ce que**
la pression dans la première colonne de distillation (3) est comprise entre 20 et 500 mbars.

15. Procédé selon la revendication 1,
**caractérisé en ce que**
le temps de séjour dans l'appareil de réaction est compris entre 0,5 et 1,5 heure.

16. Procédé selon la revendication 1,
**caractérisé en ce que**
l'évaporateur (5) est un évaporateur à film.
